# EUROPÄISCHE PATENTSCHRIFT

(11) **EP 3 955 802 B1**
(45) Veröffentlichungstag und Bekanntmachung des Hinweises auf die Patenterteilung: **06.05.2026**
(21) Anmeldenummer: 20724749.5
(22) Anmeldetag: 16.04.2020
(51) Int. Cl.: A61B 5/00, A61B 5/1455, A61B 5/024, A61B 5/0205

(54) **IN EINEN GEHÖRGANG EINSETZBARE VORRICHTUNG ZUM ERFASSEN PHYSIOLOGISCHER PARAMETER**
APPARATUS, WHICH IS INSERTABLE INTO AN EAR CANAL, FOR DETECTING PHYSIOLOGICAL PARAMETERS
DISPOSITIF POUVANT ÊTRE INSTALLÉ DANS UN CONDUIT AUDITIF POUR LA DÉTECTION DE PARAMÈTRES PHYSIOLOGIQUES

(30) Priorität: 16.04.2019 DE 102019205497
(43) Veröffentlichungstag der Anmeldung: 23.02.2022
(73) Patentinhaber: Cosinuss GmbH, 81379 München (DE)
(72) Erfinder: KREUZER, Johannes, 81377 München (DE)
(74) Vertreter: Franke, Dirk
(86) Internationale Anmeldenummer: PCT/EP2020/060699
(87) Internationale Veröffentlichungsnummer: WO 2020/212483

(56) Entgegenhaltungen:
- DE-A1- 102011 081 815
- US-A1- 2009 177 097
- US-A1- 2017 339 480

## Beschreibung

### Technisches Gebiet

Die Erfindung betrifft eine Vorrichtung zum Erfassen physiologischer Parameter gemäß dem Gegenstand des Anspruchs 1.

### Stand der Technik

Aus dem Stand der Technik sind in einen Gehörgang einsetzbare Vorrichtungen zum Erfassen physiologischer Parameter bekannt. Ebenfalls sind Vorrichtungen zum Beschallen des Trommelfells bekannt. Derartige Vorrichtungen sind z.B. tragbare Hörvorrichtungen zur Versorgung von Schwerhörigen und/oder zum Hören von Musik und/oder im Ohr zu tragende Vorrichtungen zur Bestimmung von Körpertemperatur, Herzfrequenz oder Herzfrequenzvariabilität.

Aus dem Stand der Technik bekannte, im Gehörgang zu platzierende Vorrichtungen zum Erfassen physiologischer Parameter (z.B. EP 2 717 756 B1) nutzen beispielsweise optische Messverfahren zur Erfassung der Pulsfrequenz oder der arteriellen Sauerstoffsättigung (Pulsoximetrie). Das Gewebe wird dabei mit Licht durchstrahlt, welches von monochromatischen Leuchtdioden (LED) oder Laserdioden emittiert und einer Fotodiode empfangen wird. Aus der bei definierten Wellenlängen unterschiedlichen Absorption von sauerstoffgesättigtem (arteriellem) Blut im Vergleich zu sauerstoffarmem (venösem) Blut kann die Sauerstoffsättigung ermittelt werden. Bei derartigen Messungen können aussagekräftige Messergebnisse nur dann erzielt werden, wenn die entsprechenden Sensoren (z.B. Leucht- bzw. Laserdioden, Fotodiode, Infrarotsensor) optimal im Gehörgang mit unmittelbarem Kontakt zur Innenwand des Gehörgangs positioniert sind. Zudem ist bei der Pulsoximetrie oder der Pulsfrequenz-Messung eine Positionierung erforderlich, welche sich beim Tragen des Sensors nicht oder nur geringfügig verändert. Die Sensoren sind dabei herkömmlich in einem sogenannten "Schirmchen" ("Sensorelementaufsatz") integriert, um einen optimalen Kontakt mit der Wand des Gehörgangs des Trägers zu gewährleisten. Die im Schirmchen lokalisierten Sensoren werden elektrisch kontaktiert und von anderer Stelle angesteuert. Da das Auslesen und Digitalisieren der Daten ebenfalls an anderer Stelle erfolgt, bestehen in der Regel mehrere elektrische Verbindungen zwischen den im Schirmchen lokalisierten Sensoren und einer Auswerte-Elektronik.

Bei den Vorrichtungen zum Beschallen des Trommelfells (z.B. bei Hörgeräten) werden verschiedene Bauformen unterschieden, wie z.B. Hinter-dem-Ohr-Hörgeräte (HdO), Hörgeräte mit externem Hörer (Receiver-in-the-canal-Geräte, RIC) und Indem-Ohr-Hörgeräte (IdO), wobei sich letztere je nach Tiefe der Lage im Gehörgang in verschiedene Untergruppen untergliedern (In-The-Ear, ITE; In-The-Canal, ITC; Completely-in-the-Canal, CIC; Invisible-in-the-canal, IIC). Als prinzipielle Komponenten umfassen Hörgeräte einen Schallempfänger (Eingangswandler, Mikrofon), einen Verstärker (analoger Verstärker, digitaler Signalprozessor) und einen Miniaturlautsprecher (Ausgangswandler, Hörer) zur Übertragung der Schallsignale an das Trommelfell. Der Schall wird dabei entweder mittels einer Otoplastik (Ohrstück), z.B. bei IdO Hörgeräten, oder mittels eines dünnen Schlauchs ("Slim-Tube") mit offenem Endstück ("Schirmchen", "dome"), z.B. bei HdO Hörgeräten, an das Trommelfell geleitet. Derartige Schirmchen weisen in der Regel einen oder mehrere kuppelförmig gekrümmte Ringe auf, die das Schirmchen im Gehörgang halten. Aus dem Stand der Technik bekannte Schirmchen (z.B. EP 2 360 947 A2) sind austauschbar, d. h. sie können auf das Ende eines Schallschlauchs bzw. auf einen IdO Lautsprecher aufgesteckt bzw. davon abgenommen werden, beispielsweise zur Anpassung an eine individuelle Größe des Gehörgangs oder zu Reinigungszwecken.

Bei einer in einen Gehörgang einsetzbaren, kombinierten Vorrichtung zum Beschallen des Trommelfells und zum Erfassen physiologischer Parameter ist es einerseits erforderlich, das in den Gehörgang einzusetzende Ohrstück austauschbar zu gestalten, z.B. zur Anpassung an eine individuelle Größe des Gehörgangs oder zu Reinigungszwecken, und andererseits die zuverlässige und präzise Messung der physiologischen Parameter durch optimale Positionierung der entsprechenden Sensoren im Gehörgang mit engem Kontakt zur Wand des Gehörgangs zu gewährleisten. Im Schirmchen angeordnete Sensoren können beispielsweise über elektrische Steckverbindungen mit der Auswerte-Elektronik verbunden werden. Nachteilig sind derartige, für die Anordnung im Gehörgang stark miniaturisierte elektrische Steckverbindungen sehr kostenaufwendig; weiterhin ist der Schutz dieser Verbindungen gegenüber Feuchtigkeit und Wasser technisch nur aufwendig zu realisieren.

Zudem ist in der US 2017/0339480 A1 ein optischer Herzfrequenzkopfhörer offenbart, der ein vorderes Gehäuse, eine Leiterplattenanordnung, ein hinteres Gehäuse, das an einem hinteren Ende des vorderen Gehäuses montiert ist, und einen Lichtleiter aufweist.

In der US 2009/0177097 A1 ist ein nicht-invasiver Lichtsensor zum Erfassen von Herzschlagsignalen offenbart, der ein kreisförmiges Stützelement aufweist, das in Umfangsrichtung mit einem Körperteil einer Person in Eingriff gebracht werden kann. Es ist daher die Aufgabe der vorliegenden Erfindung, eine Vorrichtung zum Erfassen physiologischer Parameter bereitzustellen, welche optimal an die individuellen anatomischen Gegebenheiten eines Gehörgangs eines Trägers angepasst ist und welche die zuverlässige, präzise Messung physiologischer Parameter erlaubt. Die Vorrichtung soll mehrfach verwendbar und kostengünstig herstellbar sein. Insbesondere ist es seine Aufgabe der vorliegenden Erfindung, eine in einen Gehörgang einsetzbare, multifunktionelle Vorrichtung bereitzustellen, welche zum Beschallen des Trommelfells und/oder zum Erfassen physiologischer Parameter eingerichtet ist. Bei der erfindungsgemäßen Vorrichtung soll ferner die Austauschbarkeit von Komponenten, insbesondere des Ohrpassstücks zu Anpassungs- und/oder Reinigungszwecken auf einfache und kostengünstige Art und Weise ermöglicht werden.

### Darstellung der Offenbarung

In einem ersten Aspekt betrifft die vorliegende Offenbarung deshalb eine Vorrichtung zum Erfassen physiologischer Parameter, die in einen Gehörgang einsetzbar ist, umfassend eine Schlauchleitung zum Einführen in den Gehörgang und ein mit der Schlauchleitung verbundenes Gehäuse. In das Gehäuse ist mindestens eine Sensoreinrichtung zum Erfassen eines physiologischen Parameters integriert. Die Schlauchleitung kann dabei entweder mit dem Gehäuse integral ausgebildet oder über eine form- und/oder reibschlüssige Verbindung mit diesem lösbar verbunden sein. Beispielsweise kann die Schlauchleitung mit dem Gehäuse über eine Rast- oder eine Drehverbindung angeordnet sein. Die Schlauchleitung umfasst an einem beim Tragen dem Trommelfell zugewandten, distalen Ende einen weitgehend zylinderförmigen Abschnitt. Der Ausdruck "weitgehend zylinderförmig" bezeichnet dabei eine Struktur in der Form eines allgemeinen Zylinders, insbesondere eines Hohlzylinders. Eine Zylindergrundfläche des genannten Abschnitts ist als geschlossene, ebene Kurve ausgebildet und unterliegt erfindungsgemäß hinsichtlich ihrer Formgebung keinen Einschränkungen; sie kann beispielsweise als Ellipse oder Kreis ausgebildet sein. Die Schlauchleitung ist am weitgehend zylinderförmigen Abschnitt des distalen Endes mit mindestens einer Sensoröffnung für mindestens eine Sensorkomponente zum Erfassen physiologischer Parameter ausgebildet. Im Zusammenhang mit der erfindungsgemäßen Vorrichtung bezieht sich der Begriff "distal" auf die Entfernung in Richtung Trommelfell des Verwenders relativ zur in das Gehäuse integrierten, mindestens einen Sensoreinrichtung der Vorrichtung. Bei einer HdO Vorrichtung liegt der zylinderförmige Abschnitt beispielsweise distal bezüglich der hinter dem Ohr liegenden Aufnahme des Gehäuses, in welcher die Sensoreinrichtung und gegebenenfalls zusätzliche Bauteile (z.B. Ladevorrichtung) angeordnet sind. Die mindestens eine Sensoröffnung ist damit an dem dem Trommelfell zugewandten und von der Sensoreinrichtung entfernten, distalen Ende angeordnet, da beim Tragen der erfindungsgemäßen Vorrichtung das distale Ende der Schlauchleitung entsprechend benachbart zum Trommelfell des Trägers zu liegen kommt. Die mindestens eine Sensoröffnung der erfindungsgemäßen Vorrichtung ist als Öffnung des distalen Endes der Schlauchleitung zur Aufnahme mindestens einer Sensorkomponente ausgebildet; in Abhängigkeit von der Dimensionierung der Sensorkomponente relativ zur Sensoröffnung können beispielsweise auch zwei oder mehrere derartige Sensorkomponenten in der Öffnung angeordnet sein. Beispielsweise können eine Fotodiode und eine LED in einer Sensoröffnung angeordnet sein, wobei in diesem Fall vorteilhaft eine optische Barriere zwischen den Sensorkomponenten angeordnet ist, um den physiologischen Parameter (z.B. Sauerstoffsättigung, Pulsfrequenz) akkurat zu erfassen. Vorteilhaft ist eine Sensorkomponente, beispielsweise eine Fotodiode oder ein Temperatursensor, jeweils in der entsprechenden Sensoröffnung derart angeordnet, dass die Sensorkomponente bündig mit der Oberfläche des weitgehend zylinderförmigen Abschnitts abschließt. Die mindestens eine Sensoröffnung ist von der Längsachse L_{A} des weitgehend zylinderförmigen Abschnitts der Schlauchleitung mit einem Abstand I_{Sensoröffnung} beabstandet. Die mindestens eine Sensoröffnung ist derart angeordnet, dass beim Tragen der Vorrichtung die mindestens eine Sensoröffnung in Kontakt zu einer Innenwand des Gehörgangs zu liegen kommt. Erfindungsgemäß können mittels einer einzigen, im Gehörgang eingesetzten Vorrichtung beispielsweise gleichzeitig zwei physiologische Parameter, z.B. die Körpertemperatur oder die Pulsfrequenz des Trägers, über entsprechende Sensorkomponenten erfasst werden. Alternativ kann ein physiologischer Parameter, beispielsweise die Pulsfrequenz des Trägers, über funktionell verschiedene Sensorkomponenten erfasst werden, wodurch die Zuverlässigkeit der Bestimmung durch redundante Messwerte erhöht wird. Bei der erfindungsgemäßen Vorrichtung ist die mindestens eine Sensorkomponente fest in der Schlauchleitung bzw. im Gehäuse verbaut und wird nicht über ein aufsteckbares Schirmchen mit der dazugehörigen, mindestens einen Sensoreinrichtung verbunden (wie bei herkömmlichen, im Ohr zu tragenden Vorrichtungen zur Erfassung physiologischer Parameter). Dadurch ist die erfindungsgemäße Vorrichtung einfach zu handhaben und erfordert keine stark miniaturisierten, im Gehörgang zu tragende Steckverbinder. Beispielsweise kann auf einfache Art und Weise eine Reinigung vorgenommen werden, ohne dass die Vorrichtung auseinander gebaut werden muss. Dadurch kann die Wiederverwertbarkeit der Vorrichtung gewährleistet werden. Vorteilhaft kann eine Multifunktionalität in einer einzigen Vorrichtung bereitgestellt werden. Durch die entsprechend exzentrische Anordnung der mindestens einen Sensorkomponente im distalen Ende des Gehäuses kann vorteilhaft einerseits die kompakte Anordnung verschiedenartiger Komponenten im räumlich abgegrenzten, distalen Endbereich der Schlauchleitung und gleichzeitig die infolge der peripheren Anordnung der in unmittelbaren Kontakt mit der Innenwand des Gehörgangs stehenden, mindestens einen Sensorkomponente optimale Signalerfassung bei wenig Störsignal ermöglicht werden.

In einer vorteilhaften Weiterbildung der Vorrichtung kann die mindestens eine Sensoröffnung von der Mantelfläche M des weitgehend zylinderförmigen Abschnitts der Schlauchleitung mit einem Abstand m_{Sensoröffnung} beabstandet sein, wobei m_{Sensoröffnung} ≤ I_{Sensoröffnung} ist. Vorteilhaft kann beispielsweise bei Vorhandensein von zwei Sensoröffnungen eine Sensoröffnung der zwei Sensoröffnungen relativ dichter an der Mantelfläche als an der Längsachse L_{A} des weitgehend zylinderförmigen Abschnitts der Schlauchleitung liegen, während eine andere Sensoröffnung mit einem sich davon unterscheidenden Abstand zur Mantelfläche angeordnet ist.

In einer weiteren Ausführungsform kann der weitgehend zylinderförmige Abschnitt des distalen Endes der Schlauchleitung mit einer Ausnehmung angeordnet sein, wobei die Ausnehmung bevorzugt eine durchgehende Ausnehmung sein kann. Der Begriff " durchgehende Ausnehmung" bezeichnet eine Ausnehmung, welche sowohl an der beim Tragen dem Trommelfell zugewandten Seite als auch an der beim Tragen vom Trommelfell abgewandten Seite "durchgehend" offen ist. Vorteilhaft kann mittels einer erfindungsgemäßen Vorrichtung, deren zylinderförmiger Abschnitt mit einer durchgehenden Ausnehmung angeordnet ist, ein physiologischer Parameter erfasst werden, ohne die Schallweiterleitung im Gehörgang zu behindern.

In einer bevorzugten Ausführungsform kann in das Gehäuse eine Hörereinrichtung zur Ausgabe von Schallsignalen integriert sein, sodass die Vorrichtung zum Beschallen des Trommelfells eingerichtet ist. Die Schlauchleitung kann dementsprechend am distalen Ende mit einer Schallaustrittsöffnung ausgebildet sein, wobei die Schallaustrittsöffnung von der Längsachse L_{A} des weitgehend zylinderförmigen Abschnitts der Schlauchleitung mit einem Abstand I_{Schallaustrittsöffnung} beabstandet sein kann. Da beim Tragen der erfindungsgemäßen Vorrichtung das distale Ende der Schlauchleitung benachbart zum Trommelfell des Trägers zu liegen kommt, können Schallwellen direkt an das Trommelfell übertragen werden. Erfindungsgemäß ist eine Schallaustrittsöffnung hinsichtlich der Längsachse des weitgehend zylindrischen Abschnitts weniger exzentrisch positioniert als die mindestens eine Sensoröffnung, diese Anordnung korrespondiert zur Anordnung der Schallaustrittsöffnung bei herkömmlichen Hörgeräten, bei welchen die Schallwellen weitgehend zentral auf das entsprechende Trommelfell geleitet werden. Somit können mittels einer einzigen, im Gehörgang eingesetzten Vorrichtung Schallwellen an das Trommelfell eines Trägers der Vorrichtung übertragen und gleichzeitig ein oder mehrere physiologische/r Parameter über entsprechende Sensorkomponenten erfasst werden.

Die Vorrichtung weist erfindungsgemäß ein Ohrpassstück zum lösbaren Anbringen an den weitgehend zylinderförmigen Abschnitt des Endes der Schlauchleitung der Vorrichtung auf, wobei das Ohrpassstück ein Mittelstück zum Eingriff in die Ausnehmung im Ende des weitgehend zylinderförmigen Abschnitts der Schlauchleitung, eine Ohrkontaktoberfläche zum Eingriff mit einer Innenwand des Gehörgangs beim Tragen und ein Endstück zum Verbinden des Mittelstücks und der Ohrkontaktoberfläche umfasst, wobei die Ohrkontaktoberfläche den weitgehend zylinderförmigen Abschnitt des Endes der Schlauchleitung wenigstens teilweise umfasst und derart daran angebracht ist, dass beim Tragen der Vorrichtung die mindestens eine Sensoröffnung in unmittelbarer Nachbarschaft zu einer Innenwand des Gehörgangs zu liegen kommt. In einer besonders bevorzugten Ausführungsform kann die Ohrkontaktoberfläche am weitgehend zylinderförmigen Abschnitt des Endes der Schlauchleitung derart angeordnet sein, dass beim Tragen der Vorrichtung die mindestens eine Sensoröffnung in Kontakt mit einer Innenwand des Gehörgangs zu liegen kommt. Erfindungsgemäß wird unter dem Begriff "Ohrpassstück" ein flexibles Ohrstück aus einem flexiblen Material verstanden, dass nicht individuell an einen Benutzer angepasst ist, sich aber aufgrund der Materialeigenschaften an die Form des Gehörgangs anpasst. Ein derartiges Ohrpassstück entspricht beispielsweise in etwa dem "Dome" (Ohrstück, Schirmchen) herkömmlicher Hörgeräte, wobei das erfindungsgemäße Ohrpassstück sich von den herkömmlichen, flexiblen "Ohrstücken" dahingehend unterscheidet, dass es derart am Ende des entsprechenden Abschnitts der Schlauchleitung angebracht ist, dass beim Tragen der Vorrichtung die mindestens eine Sensoröffnung am distalen Ende der Schlauchleitung in unmittelbarer Nachbarschaft bzw. in Kontakt zu einer Innenwand des Gehörgangs zu liegen kommt. Unter dem Ausdruck "lösbares Anbringen" wird die reibschlüssige und/oder formschlüssige Verbindung des Ohrpassstücks mit dem distalen Ende des Gehäuses verstanden, vorteilhaft können das Mittelstück des Ohrpassstücks und die Ausnehmung im distalen Ende des Gehäuses zueinander komplementär ausgebildet sein, sodass die reibschlüssige und/oder formschlüssige Verbindung zwischen dem weitgehend zylinderförmigen Abschnitt des distalen Endes der Schlauchleitung und dem Ohrpassstück sowohl über das Eingreifen des Mittelstücks in die Ausnehmung im weitgehend zylinderförmigen Abschnitt des distalen Endes der Schlauchleitung als auch über das Eingreifen der Ohrkontaktoberfläche mit dem weitgehend zylinderförmigen Abschnitt des Endes der Schlauchleitung ausgebildet wird. Die Verbindung kann gegebenenfalls durch geeignete Befestigungsmittel vor dem unbeabsichtigten Lösen gesichert werden. Das erfindungsgemäße Ohrpassstück erlaubt zum einen die einfache Anpassung an den Gehörgang des Trägers der Vorrichtung, beispielsweise über unterschiedliche Dimensionierung der Ohrkontaktoberfläche, und zum anderen die unmittelbare Nachbarschaft bzw. den direkten Kontakt einer oder mehrerer, in den entsprechenden Sensoröffnungen angeordneten Sensorkomponenten mit der Innenwand des Gehörgangs (durch entsprechendes Andrücken der entsprechenden Sensoröffnung an die Wand des Gehörgangs), wodurch die akkurate, störungsarme Erfassung physiologischer Parameter ermöglicht wird. Ein den weitgehend zylinderförmigen Abschnitt des distalen Endes der Schlauchleitung vollständig umfassendes Ohrpassstück kann im Gebrauch im Bereich der mindestens einen Sensoröffnung, z.B. im Bereich der Ohrkontaktoberfläche, aus einem transparenten Material bestehen und/oder eine im Vergleich zu der von der Sensoröffnung abgewandten Seite des Ohrpassstücks wesentlich geringere Dicke aufweisen, z.B. im Bereich der Ohrkontaktoberfläche. Ferner kann das lösbar angebrachte Ohrpassstück so auf den weitgehend zylinderförmigen Abschnitt des distalen Endes der Schlauchleitung im Sinne einer Steckverbindung aufgesteckt werden, sodass ein Auswechseln bei Verschmutzung (z.B. durch Cerumen) auf einfache Art und Weise ermöglicht wird. Demgegenüber können eine in Schlauchleitung und Gehäuse verbaute Hörereinrichtung und/oder die ebenfalls im Gehäuse verbaute, mindestens eine Sensoreinrichtung und die dazugehörige mindestens eine Sensorkomponente einfach gereinigt werden, ohne dass die elektronischen Bauteile zuvor entfernt werden müssen. Ferner kann das Ohrpassstück den Gehörgang in Bezug auf Schallwellen verschließen, d. h. dessen Schalldurchlässigkeit herabsetzen.

In einer bevorzugten Implementierung der Vorrichtung können eine Hörereinrichtung zur Ausgabe von Schallsignalen und mindestens eine Sensoreinrichtung zum Erfassen physiologischer Parameter als integrales Bauteil ausgebildet sein. In einer alternativen Implementierung können mindestens zwei Sensoreinrichtungen zum Erfassen physiologischer Parameter als integrales Bauteil ausgebildet sein. In die erfindungsgemäße Vorrichtung können so auf einfache Art und Weise unterschiedliche Kombinationen von Hörereinrichtung und Sensoreinrichtung/en eingebaut werden, beispielsweise eine Hörereinrichtung kombiniert mit einem PPG-Sensor (Photoplethysmographie-Sensor) und/oder einem Beschleunigungssensor oder eine Hörereinrichtung kombiniert mit einer Temperatursensor und/oder Infrarotsensor. Dadurch können bei größtmöglicher Flexibilität hinsichtlich der erfassten physiologischen Parameter die Herstellungskosten der Vorrichtung vorteilhaft verringert werden.

In einer bevorzugten Weiterbildung der Vorrichtung kann die Schlauchleitung mit dem Gehäuse integral ausgebildet sein. Während die Trennbarkeit der Schlauchleitung vom Gehäuse eine einfache Integration der mindestens einen Sensoreinrichtung und der mindestens einen Sensorkomponente z.B. in bestehende Vorrichtungen zum Beschallen des Trommelfells (Hörgeräte, Kopfhörer zum Musik hören) erlaubt, gewährleistet die integrale Ausführung eine einfache Handhabbarkeit z.B. beim Einsetzen/Herausnehmen oder beim Reinigen der erfindungsgemäßen Vorrichtung.

In einer weiteren Ausführungsform der Vorrichtung kann der weitgehend zylinderförmige Abschnitt am distalen Ende der Schlauchleitung mit einer konvex gewölbten Mantelfläche ausgebildet sein. Die konvexe Wölbung der Mantelfläche stellt mehr Platz bereit und erlaubt damit die Anordnung mehrerer Bauteile (Hörer, mehrere Sensorkomponenten) im distalen Ende der Schlauchleitung. Auch wird bei einem engen Gehörgang (z.B. bei Kindern) bereits das Andrücken der in der mindestens einen Sensoröffnung angeordneten Sensorkomponente an die Innenwand des Gehörgangs vereinfacht. Ferner ermöglicht die konvexe Wölbung der Mantelfläche eine verbesserte Befestigung des angebrachten Ohrpassstücks im Sinne eines verbesserten Formschlusses der Steckverbindung zwischen Ohrpassstück und distalem Ende der Schlauchleitung. In einer vorteilhaften Weiterbildung kann die Ohrkontaktoberfläche des Ohrpassstücks mit einer zum weitgehend zylinderförmigen Abschnitt des distalen Endes der Schlauchleitung weitgehend komplementären Form ausgebildet sein. Beispielsweise kann das Ohrpassstück mit seiner Ohrkontaktoberfläche ebenfalls eine weitgehend zylinderförmige Form aufweisen, wobei der Zylinder so entlang seiner Längsachse geschnitten ist, dass die mindestens eine Sensoröffnung bei aufgestecktem Ohrpassstück zu einer Innenfläche des Gehörgangs freiliegt. Das Ohrpassstück kann somit als ein Zylinderabschnitt ausgebildet sein; vorteilhaft kann das Ohrpassstück als teilweise hohler Zylinderabschnitt mit konvex gewölbter Mantelfläche ausgebildet sein. Alternativ kann das Ohrpassstück als Zylinder mit konvex gewölbter Mantelfläche ausgebildet sein, wobei die Ohrkontaktoberflächen entlang der Mantelflächen mit einer unterschiedlichen Dicke ausgebildet sind, derart, dass die Dicke im Bereich der mindestens einen Sensoröffnung geringer ist als im von der mindestens einen Sensoröffnung entfernten Bereich.

In einer besonders bevorzugten Ausführungsform der Vorrichtung kann die Längsachse Lo des Ohrpassstücks von der Längsachse L_{A} des weitgehend zylinderförmigen Abschnitts des distalen Endes der Schlauchleitung beabstandet sein. Das Ohrpassstück ist damit hinsichtlich der Längsachse des weitgehend zylindrisch ausgebildeten distalen Endes der Schlauchleitung exzentrisch angeordnet. Damit erfolgt eine Stabilisierung der mindestens einen, in der entsprechenden, mindestens einen Sensoröffnung angeordneten Sensorkomponente im Gehörgang mit optimaler Positionierung an der Innenwand des Gehörgangs zur störungsarmen Erfassung physiologischer Parameter.

In einer weiteren, besonders bevorzugten Weiterbildung der Vorrichtung kann eine Endfläche des weitgehend zylinderförmigen Abschnitts des distalen Endes der Schlauchleitung einen Radius R_{A} aufweisen und das Endstück des Ohrpassstücks als Kreis oder Kreisabschnitt mit Radius Ro ausgebildet sein, wobei R_{A}<R_{O} und der Abstand a zwischen den Mittelpunkten M_{A}, Mo der Endflächen mit 0 < a <Ro ausgebildet ist. M_{A} bezeichnet dabei den Mittelpunkt einer Endfläche des weitgehend zylinderförmigen Abschnitts des distalen Endes der Schlauchleitung und Mo den Mittelpunkt des als Kreis oder Kreisabschnitt mit Radius Ro ausgebildeten Endstücks des Ohrpassstücks. Das Ohrpassstück ist damit hinsichtlich einer Endfläche des weitgehend zylindrisch ausgebildeten, distalen Endes der Schlauchleitung exzentrisch angeordnet. Damit erfolgt eine Stabilisierung der mindestens einen, in der entsprechenden Sensoröffnung angeordneten Sensorkomponente im Gehörgang mit optimaler Positionierung an der Innenwand des Gehörgangs zur störungsarmen Erfassung physiologischer Parameter.

In einer weiteren Ausführungsform der Vorrichtung kann die Schallaustrittsöffnung weitgehend zentral in einer Endfläche des weitgehend zylinderförmigen Abschnitts des distalen Endes der Schlauchleitung angeordnet sein. Vorteilhaft kann bei der weitgehend zentralen Anordnung der Schallaustrittsöffnung auf bereits vorhandene Konstruktionen von Hörgeräten zurückgegriffen werden, in welche mindestens eine Sensoröffnung mit mindestens einer Sensoreinrichtung und der korrespondierenden, mindestens einen Sensorkomponente integriert werden kann.

In einer weiteren, bevorzugten Ausführungsform kann die Schallaustrittsöffnung dazu angepasst sein, eine Cerumenfilter-Vorrichtung aufzunehmen. Dies ist insbesondere vorteilhaft, wenn die Vorrichtung auf bereits bekannten Konstruktionen für Hörgeräte basiert.

In einer bevorzugten Weiterbildung der Vorrichtung kann das Mittelstück des Ohrpassstücks mit einer durchgehenden Ausnehmung ausgebildet sein. Unter dem Begriff "durchgehende Ausnehmung" wird eine Ausnehmung verstanden, welche sowohl an der dem Endstück zugewandten Seite als auch an der vom Endstück abgewandten Seite offen ist. Alternativ oder zusätzlich kann das Ohrpassstück mit mindestens einer Ohrpassstück-Öffnung ausgebildet sein, wobei die mindestens eine Ohrpassstück-Öffnung im Bereich der Endfläche oder im Übergang zwischen Endfläche und Ohrkontaktoberfläche des Ohrpassstücks ausgebildet sein kann. Ein Ohrpassstück mit einem derart ausgebildeten Mittelstück und/oder einer derart ausgebildeten Ohrpassstück-Öffnung erlaubt die ungestörte Schalldurchleitung, beispielsweise bei einer Vorrichtung ohne oder mit eingeschränkter Hörerfunktion. Weiterhin kann mittels eines zur Ausnehmung des Ohrpassstücks komplementär ausgebildeten Werkzeugs das einfache Anbringen und Auswechseln des Ohrpassstücks erfolgen.

In einer bevorzugten Weiterbildung der Vorrichtung kann der weitgehend zylinderförmige Abschnitt am distalen Ende des Gehäuses aus einem härteren Material bestehen als das Ohrpassstück, wobei das Ohrpassstück ein Material umfassend einen Kunststoff, ein Gummi, ein Silikon und/oder ein Elastomer aufweisen kann. Die Ausbildung der Ohrpassstücks aus einem Material mit elastischen Eigenschaften erlaubt vorteilhaft die verbesserte Verbindung zwischen distalem Ende und dem Ohrpassstück durch Reibschluss.

Wie hier verwendet, schließt die Singularform der Artikel "ein", "eine", "der", "die" und "das" die entsprechenden Mehrzahlformen ein, sofern nichts anders angegeben. Beispielsweise umfasst der Ausdruck "eine Ohrkontaktoberfläche" eine entsprechende Ohrkontaktoberfläche oder mehrere Ohrkontaktoberflächen.

Die Erfindung wird nachfolgend beispielhaft anhand der beigefügten, schematischen Zeichnungen näher erläutert. Die Zeichnungen sind nicht maßstabsgetreu; insbesondere entsprechen Verhältnisse der einzelnen Abmessungen zueinander aus Gründen der Anschaulichkeit nicht unbedingt den Abmessungsverhältnissen in tatsächlichen technischen Umsetzungen.

Die Erfindung wird durch die Ansprüche definiert.

### Kurze Beschreibung der Figuren

Figur 1 zeigt ein Ausführungsbeispiel der Vorrichtung in einer Ansicht von schräg oben, wobei ein weitgehend zylinderförmiger Abschnitt des distalen Endes der Schlauchleitung mit einer Schallaustrittsöffnung und zwei Sensoröffnungen ausgebildet ist.

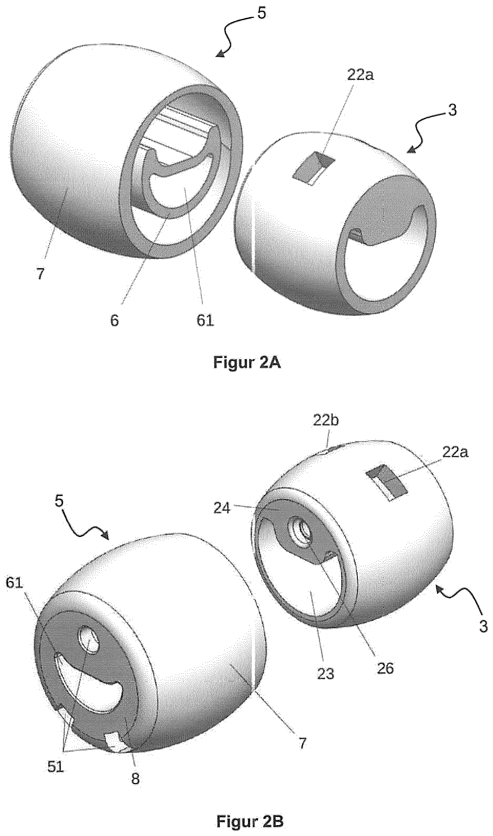

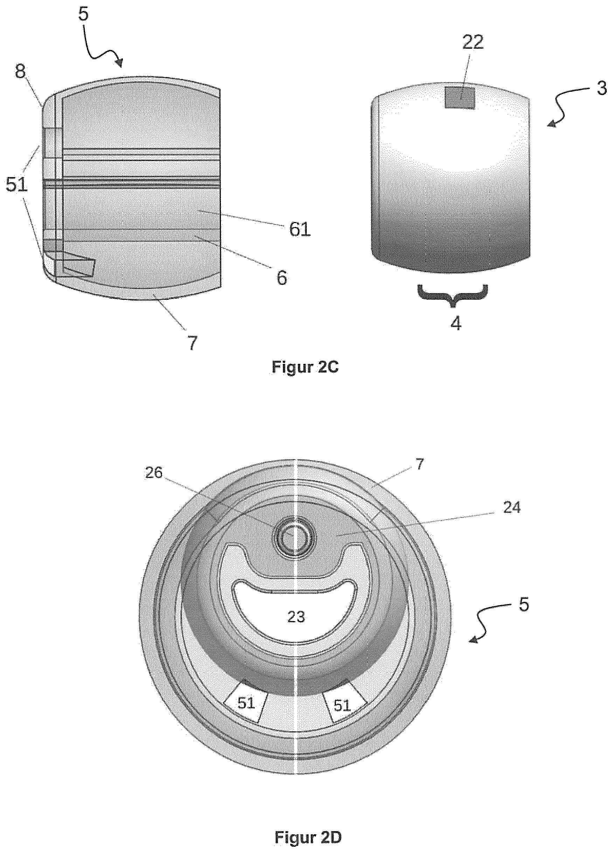

In Figur 2A ist eine Detailansicht des weitgehend zylinderförmigen Abschnitts des distalen Endes der Schlauchleitung der erfindungsgemäßen Vorrichtung dargestellt, wobei ein Ohrpassstück von dem weitgehend zylinderförmigen Abschnitt des distalen Endes der Schlauchleitung getrennt vorliegt. Figur 2B zeigt diese Anordnung in einer weiteren Ansicht, während in den Figuren 2C und 2D eine Ansicht von der Seite mit Querschnittsansicht des Ohrpassstücks (Fig. 2C) bzw. eine teilweise transparente Draufsicht auf den weitgehend zylinderförmigen Abschnitt des distalen Endes der Schlauchleitung mit aufgestecktem Ohrpassstück (Fig. 2D) dargestellt sind.
Figur 3A zeigt eine Detailansicht des distalen Endes der erfindungsgemäßen Vorrichtung in einer Ausführungsform; in Figur 3B ist eine Draufsicht auf den weitgehend zylinderförmigen Abschnitt am distalen Ende der erfindungsgemäßen Vorrichtung mit aufgestecktem Ohrpassstück gezeigt.
Die Figuren 4A bis 4C stellen ein weiteres Ausführungsbeispiel der erfindungsgemäßen Vorrichtung ohne aufgestecktes Ohrpassstück in einer Draufsicht (Fig. 4A), in einer Ansicht mit aufgestecktem Ohrpassstück von schräg unten (Fig. 4B) und in einer Draufsicht (Fig. 4C) dar.

### Bevorzugte Ausführung der Erfindung

Einander entsprechende Elemente sind in den Figuren jeweils mit den gleichen Bezugszeichen versehen.

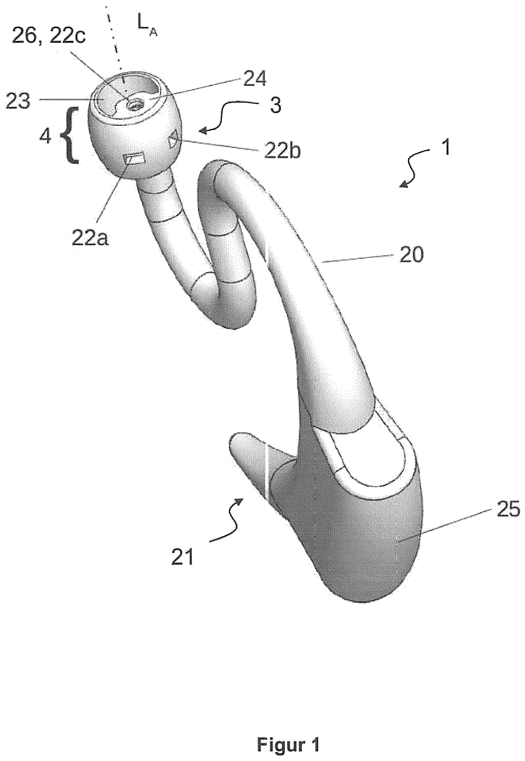

Fig. 1 zeigt ein Ausführungsbeispiel der Vorrichtung (1) in einer Ansicht von schräg oben, wobei die Vorrichtung vorliegend als HdO (Hinter-dem-Ohr) Vorrichtung ausgeführt ist. Die Vorrichtung ist zum Einsetzen in einen Gehörgang des Verwenders ausgebildet und umfasst eine Schlauchleitung (20) zum Einführen in den Gehörgang des Verwenders und ein mit der Schlauchleitung (20) verbundenes Gehäuse (21) mit einer Aufnahme (25), deren Innenraum (nicht dargestellt) zur Aufnahme verschiedener Komponenten, z.B. elektronischer, mikromechanischer oder optischer Komponenten, eingerichtet ist. In der Aufnahme (25) können beispielsweise eine Hörereinrichtung zur Ausgabe von Schallsignalen und/oder mindestens eine Sensoreinrichtung zum Erfassen physiologischer Parameter aufgenommen sein. Die Vorrichtung weist ferner ein distales Ende (3) der Schlauchleitung (20) auf, wobei sich der Begriff "distal", wie vorliegend verwendet, auf die Entfernung in Richtung Trommelfell des Verwenders relativ zur in das Gehäuse (21) integrierten Sensoreinrichtung der Vorrichtung bezieht. Bei einer HdO (Hinter-dem-Ohr) Vorrichtung liegt die Sensoreinrichtung in der Aufnahme (25) des Gehäuses (21), die beim Tragen der Vorrichtung hinter dem Ohr zu liegen kommt - das distale Ende (3) ist dementsprechend in Richtung Trommelfell angeordnet. Zwischen der Aufnahme (25) und dem distalen Ende (3) des Gehäuses (21) erstreckt sich die Schlauchleitung (20), die als aufsteckbares, dünnes Schlauchelement oder aber als S-förmiges Verbindungselement integral mit dem Gehäuse (21) und der darin ausgebildeten Aufnahme (25) ausgebildet sein, kann wie in Fig. 1 dargestellt. Beispielsweise kann durch eine flexible, S- förmige Struktur eine optimale Verspannung der Vorrichtung in definierten anatomischen Strukturen des äußeren Ohrs gewährleistet werden. Das distale Ende (3) der Schlauchleitung (20) ist mit einem weitgehend zylinderförmigen Abschnitt (4) angeordnet, der einen größeren Durchmesser aufweist als die Schlauchleitung (20). Der dargestellte weitgehend zylinderförmige Abschnitt (4) ist mit einer weitgehend kreisförmigen Grundfläche und konvex gebogenen Mantelflächen ausgebildet. Im gezeigten Ausführungsbeispiel trägt der Abschnitt eine Schallaustrittsöffnung (26) und zwei Sensoröffnungen (22a, 22b). Die Schallaustrittsöffnung (26) ist dabei in einer distalen Endfläche (24) des weitgehend zylinderförmigen Abschnitts (4) der Schlauchleitung (20) angeordnet, während die gezeigten Sensoröffnungen (22a, 22b) sich in der konvexgewölbten Mantelfläche des Abschnitts (4) befinden. Bei der in der distalen Endfläche (24) des weitgehend zylinderförmigen Abschnitts (4) der Schlauchleitung (20) angeordneten Öffnung kann es sich auch um eine weitere Sensoröffnung (22c) alternativ zur Schallaustrittsöffnung (26) handeln, beispielsweise zur Aufnahme eines Infrarotsensors, mittels dessen die Körpertemperatur über eine Infrarotemission des Trommelfells erfasst werden kann. Nach dem Einsetzen der gezeigten Vorrichtung in einen Gehörgang des Trägers ist somit die Schallaustrittsöffnung (21) in Richtung des am Ende des Gehörgangs lokalisierten Trommelfells des Verwenders ausgerichtet, während die beiden Sensoröffnungen (22a, 22b) in unmittelbarer Nähe der Innenwand des Gehörgangs zu liegen kommen. Die Sensoröffnungen (22a, 22b) stabilisieren dabei die darin angeordneten Sensorkomponenten (nicht gezeigt) zum Erfassen physiologischer Parameter, beispielsweise der Körpertemperatur und/oder der Pulsfrequenz des Verwenders, und gewährleisten deren Kontakt zur Innenwand des Gehörgangs. Die Schallaustrittsöffnung (26) und die beiden Sensoröffnungen (22a, 22b) sind von der Längsachse L_{A} (gestrichelte Linie) des weitgehend zylinderförmigen Abschnitts (4) des Gehäuses (2) jeweils mit einem Abstand I_{Schallaustrittsöffnung} bzw. einem Abstand I_{Sensoröffnung} beabstandet. Der weitgehend zylinderförmige Abschnitt (4) des distalen Endes (3) der Schlauchleitung (20) ist weiterhin mit einer Ausnehmung (23) ausgebildet. Eine durchgehende (d.h. zu beiden Zylinder-Grundflächen offene) Ausnehmung (23) kann die Weiterleitung von Schallwellen das Trommelfell selbst bei im Gehörgang eingesetzter Vorrichtung gewährleisten.

In Fig. 2A bis Fig. 2C ist jeweils eine Detailansicht des weitgehend zylinderförmigen Abschnitts (4) des distalen Endes (3) der Schlauchleitung (20) der erfindungsgemäßen Vorrichtung mit einem Ohrpassstück (5) dargestellt, wobei letzteres von Abschnitt (4) des distalen Endes (3) der Schlauchleitung (20) getrennt vorliegt (Fig. 2A -2C). Das distale Ende (3) ist mit zwei Sensoröffnungen (22a, 22b) und einer in der distalen Endfläche (24) des Gehäuses angeordneten Schallaustrittsöffnung (26) ausgebildet. Es wird deutlich, dass die Sensoröffnungen (22a, 22b) jeweils mit einem Abstand m_{Sensoröffnung} von der Mantelfläche M des weitgehend zylinderförmigen Abschnitts (4) beabstandet sind, und wobei m_{Sensoröffnung} ≤ I_{Sensoröffnung} ist. Im gezeigten Fall liegen die Sensoröffnungen direkt in der Mantelfläche des weitgehend zylinderförmigen Abschnitts (4). Weiterhin weist das distale Ende (3) der Schlauchleitung (2) eine Ausnehmung (23) auf. Diese Ausnehmung (23) ist zum Eingriff mit einem Mittelstück (6) des Ohrpassstücks (5) angeordnet. Das Mittelstück (6) des Ohrpassstücks (5) ist mit einer durchgehenden Ausnehmung (61) ausgebildet, wobei die Ausnehmung (61) zum Endstück (8) und zu der vom Endstück (8) abgewandten Seite hin offen ist, sodass ein durchgehender Raum entsteht. Das Ohrpassstück (5) weist, wie gezeigt, eine zum weitgehend zylinderförmigen Abschnitt (4) des distalen Endes (3) der Schlauchleitung (20) weitgehend komplementäre Form auf: das Mittelstück (6) kommt in der Ausnehmung (23) zu liegen, während die Ohrkontaktoberflächen (7) den Abschnitt (4) umgreifen, sodass die dem Mittelstück (6) zugewandten Seiten der Ohrkontaktoberflächen (7) an der konvex gewölbten Mantelfläche von Abschnitt (4) und die vom Mittelstück (6) abgewandten Seiten der Ohrkontaktoberflächen (7) an der Innenwand des Gehörgangs zu liegen kommen. Das Mittelstück (6) und die Ohrkontaktoberflächen (7) werden durch das Endstück (8) verbunden. Das Ohrpassstück (5) ist weiterhin mit Öffnungen (51) ausgebildet, die hier am Übergang zwischen Endstück (8) und Ohrkontaktoberflächen (7) und im Bereich der Schallaustrittsöffnung (26) angeordnet sind. Zusammen mit der im Mittelstück (6) angeordneten Ausnehmung (61) ermöglichen die Öffnungen (51) die Weiterleitung von Schallwellen an das Trommelfell bei im Gehörgang eingesetzter Vorrichtung. Vorteilhaft kann der weitgehend zylinderförmige Abschnitt (4) am distalen Ende (3) des Gehäuses (2) aus einem härteren Material bestehen als das Ohrpassstück (5). Vorteilhaft besteht das Ohrpassstück (5) aus einem flexiblen Material, beispielsweise aus Silikon, wodurch die formschlüssige Verbindung des Ohrpassstücks (5) mit dem Abschnitt (4) durch Reibschluss zusätzlich verbessert wird. Die in Fig. 2C gezeigte Querschnittsansicht des Ohrpassstücks (5) verdeutlicht, dass die Ohrkontaktoberfläche (7) des Ohrpassstücks (5) in dem Bereich, der bei der Verwendung im Bereich der Sensoröffnung (22) des weitgehend zylinderförmigen Abschnitts (4) zu liegen kommt, eine wesentlich geringere Wanddicke aufweist, als derjenige Bereich, der bei der Verwendung nicht im Bereich der Sensoröffnung (22) zu liegen kommt. Dieser Zusammenhang wird in Figur 2D noch einmal verdeutlicht, in welcher der weitgehend zylinderförmige Abschnitt (4) mit aufgestecktem, zum besseren Verständnis teilweise transparent dargestellten Ohrpassstück (5) gezeigt ist.

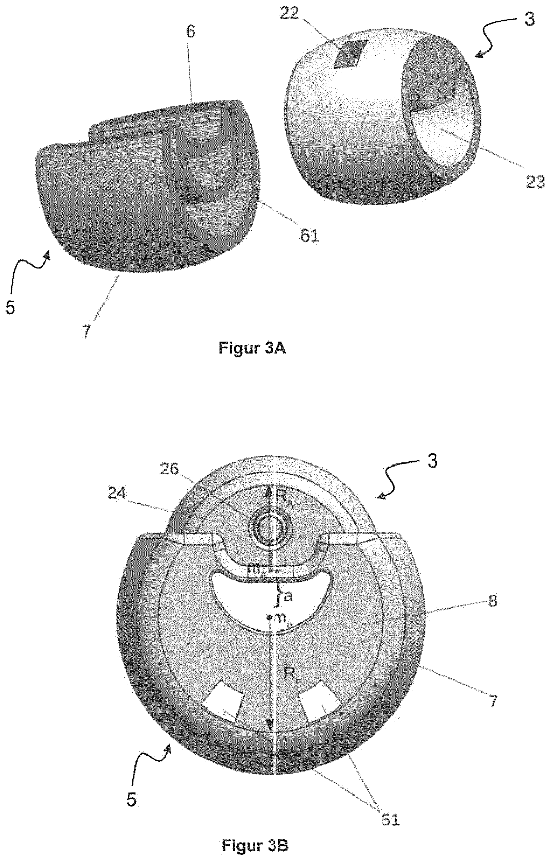

In Fig. 3A ist eine Detailansicht des distalen Endes der erfindungsgemäßen Vorrichtung in einer Ausführungsform dargestellt. Das Mittelstück (6) des Ohrpassstücks (5) ist mit einer durchgehenden Ausnehmung (61) ausgebildet, wobei die Ausnehmung (61) zum Endstück (8, in der Perspektive nicht gezeigt) und zu der vom Endstück (8) abgewandten Seite hin offen ist, sodass ein durchgehender Raum entsteht. Das Ohrpassstück (5) kann in die korrespondierende Ausnehmung (23) des weitgehend zylinderförmigen Abschnitts (4) des distalen Endes (3) der Schlauchleitung (20) eingreifen. Mit der in Fig. 3B gezeigten Draufsicht auf das distale Ende der erfindungsgemäßen Vorrichtung mit aufgestecktem Ohrpassstück (5) wird die exzentrische Anordnung des Ohrpassstücks (5) verdeutlicht. Die Endfläche (24) des weitgehend zylinderförmigen Abschnitts (4) des distalen Endes (3) der Schlauchleitung (20) weist einen Radius R_{A} auf, während das Endstück (8) des Ohrpassstücks (5) als Kreisabschnitt mit Radius Ro ausgebildet ist. Bei R_{A}<R_{O} ist Abstand a zwischen den Mittelpunkten M_{A}, Mo der Endflächen (24, 8) mit 0 < a <Ro ausgebildet. M_{A} bezeichnet dabei den Mittelpunkt der Endfläche (24) des weitgehend zylinderförmigen Abschnitts (4) des distalen Endes (3) der Schlauchleitung (20) und Mo den Mittelpunkt des als Kreisabschnitt mit Radius Ro ausgebildeten Endstücks (8) des Ohrpassstücks (5). Das Ohrpassstück (5) ist damit hinsichtlich einer Endfläche des weitgehend zylindrisch ausgebildeten distalen Endes der Schlauchleitung exzentrisch angeordnet. Wie vorliegend dargestellt, umfasst die Ohrkontaktoberfläche (7) des Ohrpassstücks (5) den weitgehend zylinderförmigen Abschnitt (4) nur teilweise; das komplementär zum weitgehend zylinderförmigen Abschnitt (4) ausgebildete Ohrpassstück (5) ist somit als ein Zylinderabschnitt ausgebildet. Erfindungsgemäß liegt das Ohrpassstück (5) exzentrisch hinsichtlich des weitgehend zylinderförmigen Abschnitts (4), so dass bei der Verwendung mindestens eine Sensoröffnung (in Fig.3A gezeigt: 22) mit einer Innenwand des Gehörgangs in Kontakt kommen kann. Die in der Sensoröffnung angeordnete und durch diese stabilisierte Sensorkomponente wird vorteilhaft durch das exzentrisch angeordnete Ohrpassstück (5) an die Gehörgangs-Innenwand angedrückt und ermöglicht so eine verbesserte Signalqualität. Aus Fig. 3A wird deutlich, dass infolge der durchgehenden Ausnehmung (61) des Mittelstücks (6) des Ohrpassstücks (5) die in einen Gehörgang des Trägers eingesetzte Vorrichtung diesen nicht schalldicht abschließt, da Schallwellen durch die entsprechende Öffnung hindurch zum Trommelfell des Trägers gelangen können. Eine oder mehrere zusätzliche Öffnungen (51) des Ohrpassstücks (5) können im Übergang von dessen Endstück (8) zur Ohrkontaktoberfläche (7) angeordnet sein und so eine weitere Schalldurchlässigkeit ermöglichen (Ansicht Fig. 3B). Diese Anordnung ist insbesondere vorteilhaft, wenn die erfindungsgemäße Vorrichtung mit mehreren Sensoreinrichtungen zur Erfassung verschiedenster physiologischer Parameter ausgebildet ist, beispielsweise zur Erfassung von Körpertemperatur, Pulsfrequenz, Sauerstoffsättigung usw. Die Sensoreinrichtungen können dabei in einem Bauteil/einer Baugruppe integral verbaut sein.

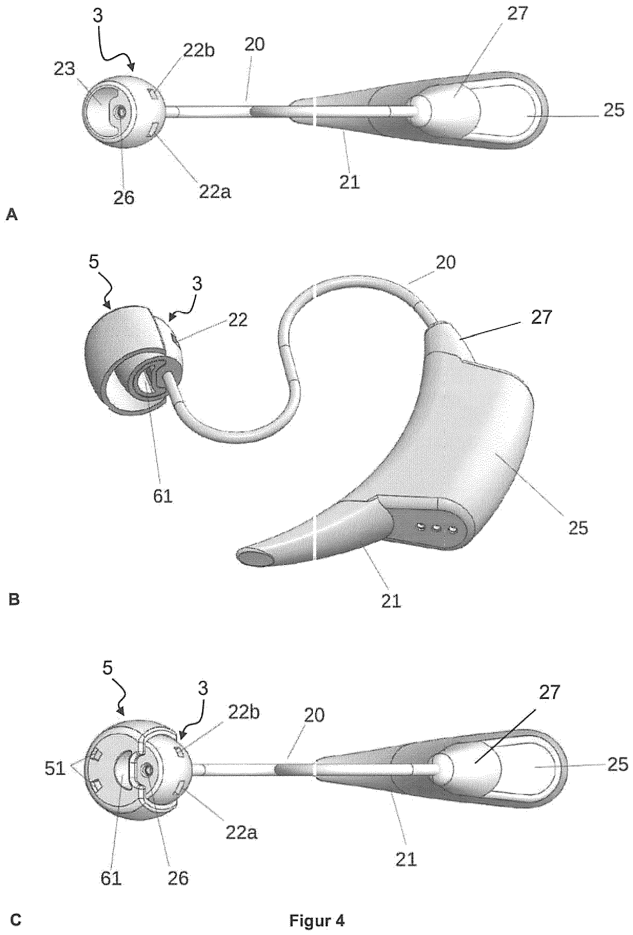

In den Fig. 4A bis 4C ist ein weiteres Ausführungsbeispiel der erfindungsgemäßen Vorrichtung in der Form eines HdO Geräts dargestellt. Fig. 4A zeigt die Vorrichtung ohne aufgestecktes Ohrpassstück in einer Draufsicht, während die Fig. 4B und 4C die Vorrichtung in einer Ansicht mit aufgestecktem Ohrpassstück von schräg unten und in einer Draufsicht (Fig. 4C) darstellen. Die Schlauchleitung (20) ist mit dem Gehäuse (21) im Bereich der Aufnahme (25) über eine Steckverbindung (27) verbunden. Die Schlauchleitung (20) ist dabei als flexibler, vorzugsweise transparenter Schlauch mit geringem Querschnitt ausgebildet, der zur Führung der entsprechenden Leitungen in den weitgehend zylinderförmigen Abschnitt (4) des distalen Endes (3) dient. Eine derart ausgebildete Schlauchleitung (20) wird z.B. bei herkömmlichen HdO Hörgeräten eingesetzt und ist vorteilhaft beim Tragen der Vorrichtung nur wenig sichtbar. Die in der Aufnahme (25) des Gehäuses (21) aufgenommenen Sensor- bzw. Hörereinrichtungen enden dabei im Bereich der Steckverbindung (27) in entsprechenden Kontakten; die in der Schlauchleitung (20) verlaufenden Leitungen enden auf der Steckverbinder-Seite in komplementären Kontakten und im Bereich des weitgehend zylinderförmigen Abschnitts in entsprechenden Sensorkomponenten bzw. Lautsprecher- Komponenten.

### Bezugszeichenliste

- 1: Vorrichtung zum Beschallen des Trommelfells und/oder zum Erfassen physiologischer Parameter
- 20: Schlauchleitung
- 21: Gehäuse
- 22: Sensoröffnung, Abschnitt 4
- 23: Ausnehmung, Abschnitt 4
- 24: Endfläche, Abschnitt 4
- 25: Aufnahme Gehäuse
- 26: Schallaustrittsöffnung
- 27: Steckverbindung
- 3: distales Ende der Schlauchleitung
- 4: weitgehend zylinderförmiger Abschnitt
- 5: Ohrpassstück
- 51: Öffnung Ohrpassstück
- 6: Mittelstück
- 61: Ausnehmung Mittelstück
- 7: Ohrkontaktoberfläche
- 8: Endstück

## Patentansprüche

1. Vorrichtung (1) zum Erfassen physiologischer Parameter, die in einen Gehörgang einsetzbar ist,
umfassend eine Schlauchleitung (20) zum Einführen in den Gehörgang und ein mit der Schlauchleitung (20) verbundenes Gehäuse (21), in das mindestens eine Sensoreinrichtung zum Erfassen eines physiologischen Parameters integriert ist,
wobei die Schlauchleitung (20), an einem beim Tragen dem Trommelfell zugewandten, distalen Ende (3) einen weitgehend zylinderförmigen Abschnitt (4) umfasst, und wobei die Schlauchleitung (20) am weitgehend zylinderförmigen Abschnitt (4) des distalen Endes (3) mit mindestens einer Sensoröffnung (22) für mindestens eine Sensorkomponente zum Erfassen physiologischer Parameter ausgebildet ist, wobei die mindestens eine Sensoröffnung (22) von der Längsachse L_{A} des weitgehend zylinderförmigen Abschnitts (4) der Schlauchleitung (20) mit einem Abstand I_{Sensoröffnung} beabstandet ist, und derart angeordnet ist, dass beim Tragen der Vorrichtung die mindestens eine Sensoröffnung (22) in Kontakt zu einer Innenwand des Gehörgangs zu liegen kommt,
**dadurch gekennzeichnet, dass** die Vorrichtung zusätzlich ein Ohrpassstück (5) zum lösbaren Anbringen an den weitgehend zylinderförmigen Abschnitt (4) des Endes (3) der Schlauchleitung (20) der Vorrichtung umfasst,
wobei das Ohrpassstück (5) ein Mittelstück (6) zum Eingriff in die Ausnehmung (23) am distalen Ende (3) des weitgehend zylinderförmigen Abschnitts (4) der Schlauchleitung (20), eine Ohrkontaktoberfläche (7) zum Eingriff mit einer Innenwand des Gehörgangs beim Tragen, und ein Endstück (8) zum Verbinden des Mittelstücks (6) und der Ohrkontaktoberfläche (7) umfasst, wobei die Ohrkontaktoberfläche (7) das Ende (3) der Schlauchleitung (20) wenigstens teilweise umfasst und derart daran angebracht ist, dass beim Tragen der Vorrichtung die mindestens eine Sensoröffnung (22) in unmittelbarer Nachbarschaft zu einer Innenwand des Gehörgangs zu liegen kommt.

2. Vorrichtung (1) gemäß Anspruch 1, wobei die mindestens eine Sensoröffnung (22) von der Mantelfläche M des weitgehend zylinderförmigen Abschnitts (4) der Schlauchleitung (20) mit einem Abstand m_{Sensoröffnung} beabstandet ist, und wobei m_{Sensoröffnung} ≤ I_{Sensoröffnung} ist.

3. Vorrichtung (1) gemäß einem der Ansprüche 1 oder 2, wobei der weitgehend zylinderförmige Abschnitt (4) des distalen Endes (3) der Schlauchleitung (20) mit einer Ausnehmung (23) angeordnet ist.

4. Vorrichtung (1) gemäß einem der vorhergehenden Ansprüche, wobei in das Gehäuse (21) eine Hörereinrichtung zur Ausgabe von Schallsignalen integriert ist, sodass die Vorrichtung zum Beschallen des Trommelfells eingerichtet ist, und wobei die Schlauchleitung (20) am distalen Ende (3) mit einer Schallaustrittsöffnung (26) ausgebildet ist, wobei die Schallaustrittsöffnung (26) von der Längsachse L_{A} des weitgehend zylinderförmigen Abschnitts (4) der Schlauchleitung (20) mit einem Abstand I_{Schallaustrittsöffnung} beabstandet ist

5. Vorrichtung (1) gemäß Anspruch 1, wobei die Ohrkontaktoberfläche (7) am weitgehend zylinderförmigen Abschnitt (4) des Endes (3) der Schlauchleitung (20) derart angeordnet ist, dass beim Tragen der Vorrichtung die mindestens eine Sensoröffnung (22) in Kontakt mit einer Innenwand des Gehörgangs zu liegen kommt.

6. Vorrichtung (1) gemäß einem der vorgehenden Ansprüche 1 bis 5, wobei die Hörereinrichtung zur Ausgabe von Schallsignalen und die mindestens eine Sensoreinrichtung zum Erfassen physiologischer Parameter als integrales Bauteil ausgebildet sind.

7. Vorrichtung (1) gemäß einem der vorhergehenden Ansprüche, wobei mindestens zwei Sensoreinrichtungen zum Erfassen physiologischer Parameter als integrales Bauteil ausgebildet sind.

8. Vorrichtung (1) gemäß einem der vorhergehenden Ansprüche, wobei die Schlauchleitung (20) mit dem Gehäuse (21) integral ausgebildet ist.

9. Vorrichtung (1) gemäß einem der vorhergehenden Ansprüche, wobei der weitgehend zylinderförmige Abschnitt (4) am distalen Ende (3) der Schlauchleitung (20) mit einer konvex gewölbten Mantelfläche M ausgebildet ist.

10. Vorrichtung (1) gemäß einem der vorhergehenden Ansprüche 1 bis 5, wobei die Ohrkontaktoberfläche (7) mit einer zum weitgehend zylinderförmigen Abschnitt (4) des distalen Endes (3) der Schlauchleitung (20) weitgehend komplementären Form ausgebildet ist.

11. Vorrichtung (1) gemäß Anspruch 10, wobei eine Längsachse Lo des Ohrpassstücks (5) von der Längsachse L_{A} des weitgehend zylinderförmigen Abschnitts (4) des distalen Endes (3) der Schlauchleitung (20) beabstandet ist.

12. Vorrichtung (1) gemäß einem der Ansprüche 10 oder 11, wobei eine Endfläche (24) des weitgehend zylinderförmigen Abschnitts (4) des distalen Endes (3) der Schlauchleitung (20) einen Radius R_{A} aufweist und das Endstück (8) des Ohrpassstücks (5) als Kreisabschnitt mit Radius Ro ausgebildet ist, wobei R_{A}<R_{O} und der Abstand a zwischen den Mittelpunkten M_{A}, Mo der Endflächen (24, 8) mit 0 < a <Ro ausgebildet ist.

13. Vorrichtung (1) gemäß einem der vorhergehenden Ansprüche 4 bis 12, wobei die Schallaustrittsöffnung (21) weitgehend zentral in einer Endfläche (24) des weitgehend zylinderförmigen Abschnitts (4) des distalen Endes (3) der Schlauchleitung (20) angeordnet ist.

## Claims

1. An apparatus (1) for detecting physiological parameters, which is insertable into an ear canal, comprising a tube (20) for insertion into the ear canal and a housing (21) connected to the tube (20), into which at least one sensor device for detecting a physiological parameter is integrated,
wherein the tube (20) at a distal end (3) facing the eardrum when worn comprises a substantially cylindrical section (4), and wherein the tubing (20) at the substantially cylindrical section (4) of the distal end (3) is provided with at least one sensor opening (22) for at least one sensor component for detecting physiological parameters,
wherein the at least one sensor opening (22) is spaced from the longitudinal axis L_{A} of the substantially cylindrical section (4) of the tube (20) by a distance I_{sensoropening} and is arranged such that, when the apparatus is worn, the at least one sensor opening (22) comes into contact with an inner wall of the ear canal,
**characterized in that** the apparatus further comprises an ear fitting (5) for detachably attaching to the substantially cylindrical section (4) of the end (3) of the tube (20),
wherein the ear fitting (5) comprises a central piece (6) for engagement with a recess (23) at the distal end (3) of the substantially cylindrical section (4) of the tube (20), an ear contact surface (7) for engagement with an inner wall of the ear canal when worn, and an end piece (8) for connecting the central piece (6) and the ear contact surface (7),
wherein the ear contact surface (7) at least partially surrounds the end (3) of the tube (20) and is arranged such that, when the apparatus is worn, the at least one sensor opening (22) comes to lie in immediate proximity to an inner wall of the ear canal.

2. The apparatus (1) according to claim 1, wherein the at least one sensor opening (22) is spaced from the mantle surface M of the substantially cylindrical section (4) of the tube (20) by a distance m_{sensoropening}, and wherein m_{sensoropening} ≤ I_{sensoropening}.

3. The apparatus (1) according to any one of claims 1 or 2, wherein the substantially cylindrical section (4) of the distal end (3) of the tube (20) is provided with a recess (23).

4. The apparatus (1) according to any one of the preceding claims, wherein a hearing device for outputting acoustic signals is integrated into the housing (21), such that the apparatus is adapted to provide sound to the eardrum, and wherein the tube (20) at the distal end (3) is provided with a sound outlet opening (26),
wherein the sound outlet opening (26) is spaced from the longitudinal axis L_{A} of the substantially cylindrical section (4) of the tubing (20) by a distance I_{soundoutletopening}.

5. The apparatus (1) according to claim 1, wherein the ear contact surface (7) on the substantially cylindrical section (4) of the end (3) of the tube (20) is arranged such that, when the apparatus is worn, the at least one sensor opening (22) comes into contact with an inner wall of the ear canal.

6. The apparatus (1) according to any one of claims 1 to 5, wherein the hearing device for outputting acoustic signals and the at least one sensor device for detecting physiological parameters are formed as an integral component.

7. The apparatus (1) according to any one of the preceding claims, wherein at least two sensor devices for detecting physiological parameters are formed as an integral component.

8. The apparatus (1) according to any one of the preceding claims, wherein the tube (20) is formed integrally with the housing (21).

9. The apparatus (1) according to any one of the preceding claims, wherein the substantially cylindrical section (4) at the distal end (3) of the tube (20) is provided with a convexly curved mantle surface M.

10. The apparatus (1) according to any one of claims 1 to 5, wherein the ear contact surface (7) is formed with a shape substantially complementary to the substantially cylindrical section (4) of the distal end (3) of the tube (20).

11. The apparatus (1) according to claim 10, wherein a longitudinal axis Lo of the ear fitting (5) is spaced from the longitudinal axis L_{A} of the substantially cylindrical section (4) of the distal end (3) of the tube (20).

12. The apparatus (1) according to any one of claims 10 or 11, wherein an end face (24) of the substantially cylindrical section (4) of the distal end (3) of the tube (20) has a radius R_{A} and the end piece (8) of the ear fitting (5) is formed as a circular segment with radius Ro,
wherein R_{A} < Ro and the distance a between the centers M_{A}, Mo of the end faces (24, 8) is formed such that 0 < a < Ro.

13. The apparatus (1) according to any one of the preceding claims 4 to 12, wherein the sound outlet opening (21) is arranged substantially centrally in an end face (24) of the substantially cylindrical section (4) of the distal end (3) of the tube (20).

## Revendications

1. Dispositif (1) pour détecter des paramètres physiologiques, pouvant être inséré dans un conduit auditif, comprenant un tube (20) destiné à être introduit dans le conduit auditif et un boîtier (21) relié au tube (20), dans lequel au moins un dispositif capteur pour détecter un paramètre physiologique est intégré,
le tube (20) comprenant, à une extrémité distale (3) tournée vers le tympan lors du port, une section sensiblement cylindrique (4), et le tube (20) étant formé à la section sensiblement cylindrique (4) de l'extrémité distale (3) avec au moins une ouverture de capteur (22) pour au moins un composant capteur destiné à détecter des paramètres physiologiques, ladite au moins une ouverture de capteur (22) étant espacée de l'axe longitudinal L_{A} de la section sensiblement cylindrique (4) du tube (20) d'une distance I_{ouverturedecapteur} et étant disposée de telle manière que, lors du port du dispositif, ladite au moins une ouverture de capteur (22) entre en contact avec une paroi interne du conduit auditif,
**caractérisé en ce que** le dispositif comprend en outre un embout auriculaire (5) destiné à être monté de manière détachable sur la section sensiblement cylindrique (4) de l'extrémité (3) du tube (20),
l'embout auriculaire (5) comprenant une pièce centrale (6) pour s'engager dans la cavité (23) à l'extrémité distale (3) de la section sensiblement cylindrique (4) du tube (20), une surface de contact auriculaire (7) pour s'engager avec la paroi interne du conduit auditif lors du port, et une pièce terminale (8) pour relier la pièce centrale (6) et la surface de contact auriculaire (7), la surface de contact auriculaire (7) englobant au moins partiellement l'extrémité (3) du tube (20) et étant disposée de telle manière que, lors du port du dispositif, ladite au moins une ouverture de capteur (22) se trouve à proximité immédiate d'une paroi interne du conduit auditif.

2. Dispositif (1) selon la revendication 1, ladite au moins une ouverture de capteur (22) étant espacée de la surface latérale M de la section sensiblement cylindrique (4) du tube (20) d'une distance m_{ouverturedecapteur}, et m_{ouverturedecapteur} ≤ louverturedecapteur.

3. Dispositif (1) selon l'une des revendications 1 ou 2, la section sensiblement cylindrique (4) de l'extrémité distale (3) du tube (20) étant pourvue d'une cavité (23).

4. Dispositif (1) selon l'une des revendications précédentes, le boîtier (21) comprenant un dispositif auditif intégré pour délivrer des signaux sonores, de sorte que le dispositif est adapté à délivrer un son au tympan, le tube (20) étant formé à l'extrémité distale (3) avec une ouverture de sortie sonore (26), l'ouverture de sortie sonore (26) étant espacée de l'axe longitudinal L_{A} de la section sensiblement cylindrique (4) du tube (20) d'une distance I_{ouverturedesortiesonore}.

5. Dispositif (1) selon la revendication 1, la surface de contact auriculaire (7) étant disposée sur la section sensiblement cylindrique (4) de l'extrémité (3) du tube (20) de telle manière que, lors du port du dispositif, ladite au moins une ouverture de capteur (22) se trouve en contact avec une paroi interne du conduit auditif.

6. Dispositif (1) selon l'une des revendications 1 à 5, le dispositif auditif pour délivrer des signaux sonores et ledit au moins un dispositif capteur pour détecter des paramètres physiologiques étant formés en tant qu'élément intégré.

7. Dispositif (1) selon l'une des revendications précédentes, au moins deux dispositifs capteurs pour détecter des paramètres physiologiques étant formés en tant qu'élément intégré.

8. Dispositif (1) selon l'une des revendications précédentes, le tube (20) étant formé de manière intégrale avec le boîtier (21).

9. Dispositif (1) selon l'une des revendications précédentes, la section sensiblement cylindrique (4) à l'extrémité distale (3) du tube (20) étant formée avec une surface latérale M convexe.

10. Dispositif (1) selon l'une des revendications 1 à 5, la surface de contact auriculaire (7) étant formée avec une forme sensiblement complémentaire à la section sensiblement cylindrique (4) de l'extrémité distale (3) du tube (20).

11. Dispositif (1) selon la revendication 10, un axe longitudinal Lo de l'embout auriculaire (5) étant espacé de l'axe longitudinal L_{A} de la section sensiblement cylindrique (4) de l'extrémité distale (3) du tube (20).

12. Dispositif (1) selon l'une des revendications 10 ou 11, une face terminale (24) de la section sensiblement cylindrique (4) de l'extrémité distale (3) du tube (20) ayant un rayon R_{A}, et la pièce terminale (8) de l'embout auriculaire (5) étant formée en tant que segment circulaire de rayon Ro, R_{A} < Ro et la distance a entre les centres M_{A}, Mo des faces terminales (24, 8) étant telle que 0 < a < Ro.

13. Dispositif (1) selon l'une des revendications 4 à 12, l'ouverture de sortie sonore (21) étant disposée sensiblement au centre d'une face terminale (24) de la section sensiblement cylindrique (4) de l'extrémité distale (3) du tube (20).
